# EUROPEAN PATENT SPECIFICATION

(11) **EP 2 145 962 B1**
(45) Date of publication and mention of the grant of the patent: **16.11.2016**
(21) Application number: 09170310.8
(22) Date of filing: 14.07.2006
(51) Int. Cl.: A61K 38/04, C07K 5/12, C12P 21/02

(54) **Process for the manufacture of eptifibatide**
Verfahren zur Herstellung von Eptifibatid
Processus de fabrication d'eptifibatide

(30) Priority: 15.07.2005 US 699818 P
(43) Date of publication of application: 20.01.2010
(62) Divisional of application: 06777777.1
(73) Proprietor: PEPTISYNTHA SA, 1120 Brussels (BE)
(72) Inventor: Collin, André, 1081 Brussels (BE); Tambuyser, Thierry, 1120 Brussels (BE)
(74) Representative: Office Kirkpatrick

(56) References cited:
- WO-A-02/072132
- WO-A-03/093302
- WO-A-2005/100381
- WO-A-2006/041945
- WO-A-2006/119388
- US-A- 5 686 571
- US-A- 5 747 447
- US-A- 5 795 868
- "MILLENIUM PHARMACEUTICALS INC URL - www.fda.gov/medwatch/SAFETY/2004/julPI/Int eegrilin_PI.pdf" INTERNET ARTICLE, [Online] April 2004 (2004-04), XP002304465 Retrieved from the Internet: URL:www.fda.gov/medwatch/SAFETY/2004/julPI /Inteegrilin_PI.pdf> [retrieved on 2004-08-11]
- SCARBOROUGH R M ET AL: "DESIGN OF POTENT AND SPECIFIC INTEGRIN ANTAGONISTS. ÖPEPTIDE ANTAGONISTS WITH HIGH SPECIFICITY FOR GLYCOPROTEIN IIB-IIIA" JOURNAL OF BIOLOGICAL CHEMISTRY, AMERICAN SOCIETY OF BIOLOCHEMICAL BIOLOGISTS, BIRMINGHAM,, US, vol. 268, no. 2, 15 January 1993 (1993-01-15), pages 1066-1073, XP000336707 ISSN: 0021-9258
- PHILLIPS DAVID R ET AL: "Clinical pharmacology of eptifibatide" AMERICAN JOURNAL OF CARDIOLOGY, vol. 80, no. 4A, 1997, pages 11B-20B, XP009072969 ISSN: 0002-9149
- BAJUSZ S ET AL: "FURTHER ENHANCMENT OF ANALGESIC ACTIVITY: ENKEPHALIN ANALOGS WITH TERMINAL GUANIDINO GROUP" FEBS LETTERS, ELSEVIER, AMSTERDAM, NL, vol. 110, no. 1, 1 January 1980 (1980-01-01), pages 85-87, XP002910916 ISSN: 0014-5793
- ANDERLUH M ET AL: "Design and synthesis of novel platelet fibrinogen receptor antagonists with 2H-1,4-benzoxazine-3(4H)-one scaffold. A systematic study" EUROPEAN JOURNAL OF MEDICINAL CHEMISTRY, EDITIONS SCIENTIFIQUE ELSEVIER, PARIS, FR, vol. 40, no. 1, 1 January 2005 (2005-01-01), pages 25-49, XP025379811 ISSN: 0223-5234 [retrieved on 2005-01-01]
- GUZZETTA, ANDREW: "Reverse Phase HPLC Basics for LC/MS" IONSOURCE, [Online] 22 July 2001 (2001-07-22), pages 1-9, XP002556091 Internet Retrieved from the Internet: URL:http://ionsource.com/tutorial/chromato graphy/rphplc.htm> [retrieved on 2009-11-18]
- Luwei Zhao ET AL: "Stabilization of eptifibatide by cosolvents", International Journal of Pharmaceutics, vol. 218, no. 1-2, 1 May 2001 (2001-05-01) , pages 43-56, XP055119808, ISSN: 0378-5173, DOI: 10.1016/S0378-5173(01)00618-4
- David Carr: "The Handbook of Anylysis and Purification of peptides and proteins by Reversed-Phase HPLC", 2002 pages 1-70,

## Description

The present invention relates to a process for the manufacture of eptifibatide, in particular to such a process comprising a purification step.

Eptifibatide, which is commercialised by MILLENNIUM PHARMACEUTICALS under the name Integrelin™ is a cyclical heptapeptide of sequence 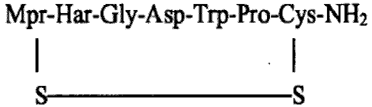 that selectively blocks the platelet glycoprotein IIb/ IIIa receptor. It reversibly binds to platelets and has a short half-life. It has demonstrated efficacy as an intravenous solution in the treatment of patients during coronary angioplasty, myocardial infarction and angina.

Callens discloses in the IBC talk, San Diego, 1999 a method of manufacturing eptifibatide.

Scarborough et al. US 5,686,571 discloses laboratory-scale chromatography of eptifibatide by reversed phase HPLC using a gradient of acetonitrile in trifluoroacetic acid, wherein the chromatography fractions are directly lyophilized.
Scarborough also discloses the design of potent and specific integrin antagonists, especially peptide antagonists with high specificity for glycoprotein IIB-IIIA (see Scarborough et al.: "Design of potent and Specific Integrin Antagonists. PEPTIDE ANTAGONISTS WITH HIGH SPECIFICITY FOR GLYCOPROTEIN IIB-IIIA; Journal of Biological Chemistry, American Societey of Biochemical Biologists, Birmingham, US, vol. 268, no.2, 15 January 1993 (1993-01-15), pages 1066-1073, XP000336707 ISSN: 021-9258). Furthermore, Scarborough et al. US 5,795,868 discloses peptides useful in inhibiting platelet aggregation. These peptides contain the binding sequence Har-G-D and are disulfide-bridged cyclic compounds. Zhao et al., 2011 "Stabilization of eptifibatide by cosolvents"; International Journal of Pharmaceutics, Vol. 218, 43-56 discloses long term improvement of a 0.2% eptifibatide solutions by putting eptifibatide powders in solution.
Finally, Swift et al. US 5,747,447 discloses an injectable biologically active polypeptide which is stabilized by dissolving said polypeptide forming a liquid solution in citrate buffer of about pH 5.0-5.5.

It was desirable to have available a process which allows for manufacture of eptifibatide having required purity for its applications and which presents advantages in terms of productivity and required manufacturing equipment.

In consequence, the invention concerns a process for manufacturing eptifibatide which comprises subjecting a solution of eptifibatide derivative in a solvent to a concentration step, wherein the pH of said solution is between 3 and 5.

It has been found, surprisingly, that the process according to the invention allows to provide concentrated pure eptifibatide material without substantial formation of by-products, in particular dimerization of eptifibatide, during the concentration step. It also allows in particular to meet specifications concerning purification related organic impurities such as for example organic solvents, in particular acetonitrile.

In the present invention, "solution of eptifibatide derivative" is intended to denote a substantially homogeneous, preferably a totally homogeneous liquid phase containing eptifibatide derivative dissolved therein.

In the present invention, "eptifibatide derivative" is intended to denote the acetate salt form of the cyclic heptapeptide, which is the commercialized drug.

In the process according to the invention the pH of the solution is less than or equal to 5 and preferably less than or equal to about 4.6. In the process according to the invention, the pH of the solution is more than or equal to 3 and preferably more than or equal to 4.

In the process according to the invention the pH of the solution is preferably maintained at the values indicated above throughout the concentration step. It is not preferred to work outside the indicated values though it might be possible to do so for a short period of time, without materially affecting the results obtained by virtue of the teaching of the present invention.

In the process according to the invention the pH of the solution is maintained within a given interval around a chosen target pH value, as described above. Preferably, this interval is about ±0.5. The pH is generally maintained by measuring and adjusting, if necessary, by addition of pH controlling agent. A preferred pH controlling agent is an acetate buffer, for example in particular a mixture of eptifibatide acetate and suitably controlled amount of acetic acid. The pH controlling agent can be added to the solution before or during the concentration step.

In the process according to the invention, the concentration step is generally carried out at a temperature less than or equal to 40°C preferably less than or equal to 35°C and more preferably less than or equal to 30°C. In the process according to the invention, the concentration step is generally carried out at a temperature higher than or equal to -20°C preferably higher than or equal to 0°C and more preferably higher than or equal to 20°C. A concentration step carried out at a temperature of about 30°C is more particularly preferred.

In the process according to the invention, the temperature is preferably maintained at the values indicated above throughout the concentration step. It is not preferred to work outside the indicated values though it might be possible to do so for a short period of time, without materially affecting the results obtained by virtue of the teaching of the present invention.

In the process according to the invention the temperature during the concentration step is preferably maintained within a given interval around a chosen target temperature value, as described above. Preferably, this interval is about ±5°C and more preferably ±2°C.

In a particular embodiment of the process according to the invention the solvent comprises water and a polar organic co-solvent. Examples of polar organic co-solvent include organic nitriles and alcohols. Organic nitriles are preferred, acetonitrile is more particularly preferred. Among alcohols, alkyl alcohols, for example C1-C4 alcohols are preferred. Methanol and ethanol, in particular methanol can be suitably used.

In this embodiment the volume ratio of water to polar organic co-solvent is generally equal to or higher than 50:50 relative to the mixture water/ polar organic co-solvent. Preferably this ratio is equal to or higher than 70:30. In this embodiment, the volume ratio of water to polar organic co-solvent is generally equal to or lower than 90:10 relative to the mixture water/ polar organic co-solvent. Preferably this ratio is equal to or lower than about 80:20.

In this process according to the invention, the solvent comprises acetic acid. When the solvent contains water and a polar organic co-solvent, the acetic acid content is preferably 0.1-1 volume parts per 100 volume parts of water/polar organic co-solvent mixture and more preferably about 0.3 volume parts per 100 volume parts of water/ polar organic co-solvent mixture.

In the process according to the invention, the eptifibatide derivative contained in the solution consists essentially of eptifibatide acetate.

In the process according to the invention, the purity of the eptifibatide derivative in the solution subjected to the concentration operation is generally at equal to or higher than 98.5 area % determined by HPLC. Preferably this purity is equal to or higher than 99 area % and more preferably equal to or higher than 99.5 area %. Generally, the purity of the eptifibatide derivative in the solution subjected to the concentration operation does not exceed 99.7 area % determined by HPLC.

In the process according to the invention, the eptifibatide derivative concentration in the solution subjected to the concentration operation is equal to or higher than 0.5 wt. %, preferably equal to or higher than 1 wt. % and more preferably equal to or higher than 2 wt. %. In the process according to the invention, the eptifibatide derivative concentration in the solution subjected to the concentration operation is lower than 3 wt. %.

In the process according to the invention, the concentration step is frequently selected from an evaporation step, a reversed osmosis step and a precipitation or crystallization step. Preferably, the concentration step is selected from an evaporation step and a reversed osmosis step, and more preferably it is an evaporation step.

In particular when the concentration step is selected from an evaporation step and a reversed osmosis step, the material recovered from the concentration operation is generally a concentrated solution of eptifibatide derivative.

In this embodiment, the concentrated eptifibatide derivative solution recovered from the concentration operation has preferably an eptifibatide derivative concentration below the solubility limit of the eptifibatide derivative in the solvent having been subjected to evaporation or reversed osmosis, at the temperature of the concentration step. Preferably, the solution has a concentration of at least 70 % molar of the solubility limit of the eptifibatide derivative in the solvent having been subjected to evaporation or reversed osmosis, at the temperature of the concentration step, more preferably at least 75 % molar and most preferably about 80 % molar of the solubility limit. It generally does not exceed about 85 % molar of the solubility limit. It has been found that limiting the concentration step to this value allows for improved control of the process, thus avoiding undesired precipitation and associated loss of product lots. In a less advantageous embodiment, the eptifibatide derivative concentration is substantially below that solubility limit.

When a mixture of water and acetonitrile is used, the concentrated eptifibatide derivative solution has an eptifibatide derivative concentration equal to or lower than the about 5 wt. % and equal to or higher than 4 wt. %.

In the process according to the invention, the purity of the eptifibatide derivative in the concentrated material recovered from the concentration operation is generally at equal to or higher than 98.5 area % determined by HPLC. Preferably this purity is equal to or higher than 99 area % and more preferably equal to or higher than 99.5 area %. Generally, the purity of the eptifibatide derivative in the concentrated material recovered from the concentration operation does not exceed 99.7 area % determined by HPLC.

In the process according to the invention, the content of dimeric eptifibatide derivatives in the concentrated material recovered from the concentration operation is generally equal to or lower than 0.2 area % determined by HPLC, preferably equal to or lower than 0.1 area %. If dimeric eptifibatide derivatives are present in the concentrated material recovered from the concentration operation, their concentration can be at least 0.05 area % determined by HPLC.

In a preferred embodiment of the process according to the invention, the concentration step comprises evaporating solvent from the solution. In this embodiment, the solvent is generally evaporated at a pressure higher than or equal to 0.001 bars, preferably higher than or equal to 0.01 bars. In this embodiment, the solvent is generally evaporated at a pressure lower than or equal to 1 bar, preferably lower than or equal to 0.5 bars, more preferably lower than or equal to 0.1 bars.

When a mixture of water and acetonitrile is used in this preferred embodiment, the evaporation comprises evaporating a mixture of water and acetonitrile, whereby the concentration of acetonitrile in the solution is reduced.

In a particularly preferred aspect of this embodiment, water is added to the solution during the evaporation step so as to further reduce the content and preferably substantially eliminate acetonitrile in the concentrated solution. Typical acetonitrile contents in the concentrated solution are less than about 100 mg/kg, preferably less or equal than about 30 mg/kg and most preferably less or equal than about 20 mg/kg.

In a preferred embodiment, the process according to the invention comprises steps (a) to (d) wherein
(a) crude eptifibatide derivative is manufactured by coupling of peptide fragments, for example according to the reaction sequence disclosed in Callens, IBC Talk cited above;
(b) the crude eptifibatide derivative is subjected to a purification operation comprising at least one liquid chromatography operation to obtain a solution of eptifibatide derivative;
(c) the solution of eptifibatide derivative is subjected to the concentration step according the invention to obtain material having increased concentration of eptifibatide; and
(d) the material having increased concentration of eptifibatide is subjected to a lyophilization operation to obtain eptifibatide.

The example here after is intended to illustrate the invention without however limiting its scope.

### Example

80l of a solution of eptifibatide acetate having a purity of 99.5 % measured by analytical HPLC on a C-18 reversed phase column, having an eptifibatide concentration of 15.2 g/l in a mixture water/acetonitrile/acetic acid 70 parts by volume/30 parts by volume/0.3 parts by volume said solution having a pH of 4.6 was introduced into a tubular climbing film evaporator and an acetonitrile/water mixture was evaporated at a temperature kept at about 30°C. During the evaporation, 30 l of water were fed continuously at the rate of evaporation. The evaporation was then continued until the volume of the solution had decreased to about 30l. The pH of this solution was 4.2. A concentrated solution of eptifibatide acetate having a concentration of 40.5 g/l a and purity of 99.5 % and no detectable dimer was recovered and introduced into a lyophilization step. Eptifibatide acetate having appropriate purity for use in medicament was obtained in high yield from that step.

The process according to the invention allows to reduce the volume of solution introduced into further isolation procedures such as cost-intensive lyophilization step while maintaining required purity of eptifibatide derivative for pharmaceutical application and substantially avoiding formation of by-products, in particular dimeric by-products.

## Claims

1. A solution of eptifibatide acetate in a solvent comprising water, a polar organic co-solvent and acetic acid wherein the concentration of the eptifibatide acetate in said solution is equal to or higher than 0,5 % wt. and equal to or lower than 3 % wt. of eptifibatide acetate, wherein the pH of said solution is from 3 to 5.

2. The solution according to claim 1, wherein the volume ratio of water to polar organic co-solvent is equal to or higher than 70:30.

3. The solution according to claim 1 or 2, which contains from 0,1 to 1 volume part of acetic acid per 100 volume parts of mixture of water/polar organic co-solvent.

4. The solution according to any one of the preceding claims 1 to 3, wherein the polar organic co-solvent is acetonitrile.

5. Use of the solution according to any one of claims 1 to 4 in a manufacturing process of eptifibatide which comprises subjecting a solution of eptifibatide acetate in a solvent to a concentration step, wherein the pH of said solution is less than or equal to 7.

6. A process for manufacturing eptifibatide wherein the solution according to any one of claims 1 to 4 is used in a concentrating step to obtain a concentrated eptifibatide acetate material.

7. The process according to claim 6, wherein the concentrated eptifibatide acetate material is a solution.

8. The process according to claim 7, wherein the concentrated solution has an eptifibatide acetate concentration of from 4 to 5 wt. %.

9. The process according to anyone of claims 6 to 8, wherein the concentration step is carried out at a temperature less than or equal to 40°C.

10. The process according to claim 9, wherein the temperature is from 0 to 35 °C.

11. The process according to claim 10, wherein the temperature is from 20 to 35 °C.

12. The process according to anyone of claims 6 to 11, wherein the concentration step comprises evaporating solvent from the solution.

13. The process according to claim 12, wherein the solvent is evaporated at a pressure from 0.001 to 0.5 bars.

14. Concentrated solution of eptifibatide acetate in a solvent comprising water, acetonitrile and acetic acid wherein the concentration of the eptifibatide acetate in said solution is equal to or higher than 4% wt. and equal to or lower than 5 % wt. of eptifibatide acetate, wherein the pH of the said concentrated solution of eptifibatide is from 3 to 5.

## Patentansprüche

1. Lösung von Eptifibatid-Acetat in einem Lösungsmittel, umfassend Wasser, ein polares organisches Zusatzlösungsmittel und Essigsäure, wobei die Konzentration des Eptifibatid-Acetats in der Lösung gleich oder höher als 0,5 Gew.-% und gleich oder niedriger als 3 Gew.-% Eptifibatid-Acetat ist, wobei der pH der Lösung von 3 bis 5 ist.

2. Lösung nach Anspruch 1, wobei das Volumenverhältnis von Wasser zu polarem organischen Zusatzlösungsmittel gleich oder größer als 70:30 ist.

3. Lösung nach Anspruch 2 oder 3, die von 0,1 bis 1 Volumenanteil Essigsäure pro 100 Volumenanteile Mischung aus Wasser/polarem organischen Zusatzlösungsmittel enthält.

4. Lösung nach einem der vorstehenden Ansprüche 1 bis 3, wobei das polare organische Zusatzlösungsmittel Acetonitril ist.

5. Verwendung der Lösung nach einem der Ansprüche 1 bis 4 in einem Herstellungsprozess von Eptifibatid, der das Behandeln einer Eptifibatid-Acetat-Lösung in einem Lösungsmittel mit einem Konzentrationsschritt umfasst, wobei der pH der Lösung kleiner als oder gleich 7 ist.

6. Herstellungsprozess für Eptifibatid, wobei die Lösung nach einem der Ansprüche 1 bis 4 in einem Konzentrationsschritt verwendet wird, um ein konzentriertes Eptifibatid-Acetat-Material zu erhalten.

7. Prozess nach Anspruch 6, wobei das konzentrierte Eptifibatid-Acetat-Material eine Lösung ist.

8. Prozess nach Anspruch 7, wobei die konzentrierte Lösung eine Eptifibatid-Acetat-Konzentration von 4 Gew.-% bis 5 Gew.- % aufweist.

9. Prozess nach einem der Ansprüche 6-8, wobei der Konzentrationsschritt bei einer Temperatur von weniger als oder gleich 40 °C durchgeführt wird.

10. Prozess nach Anspruch 9, wobei die Temperatur von 0 °C bis 35 °C ist.

11. Prozess nach Anspruch 10, wobei die Temperatur von 20 °C bis 35 °C ist.

12. Prozess nach einem der Ansprüche 6-11, wobei der Konzentrationsschritt das Verdunsten von Lösungsmittel aus der Lösung umfasst.

13. Prozess nach Anspruch 12, wobei das Lösungsmittel bei einem Druck von 0,001 bar bis 0,5 bar verdunstet wird.

14. Konzentrierte Lösung von Eptifibatid-Acetat in einem Lösungsmittel, umfassend Wasser, Acetonitril und Essigsäure, wobei die Konzentration des Eptifibatid-Acetats in der Lösung gleich oder größer als 4 Gew.-% und gleich oder geringer als 5 Gew.-% Eptifibatid-Acetat ist, wobei der pH der konzentrierten Eptifibatid-Lösung von 3 bis 5 ist.

## Revendications

1. Une solution d'acétate d'eptifibatide dans un solvant comprenant de l'eau, un co-solvant organique polaire et de l'acide acétique dans laquelle la concentration de l'acétate d'eptifibatide dans ladite solution est égale à ou supérieure à 0,5 % en poids et égale ou inférieure à 3 % en poids d'acétate d'eptifibatide, dans laquelle le pH de ladite solution varie de 3 à 5.

2. La solution selon la revendication 1, dans laquelle le ratio volumique d'eau au co-solvant organique polaire est égale à ou supérieure à 70:30.

3. La solution selon la revendication 1 ou 2, qui contient de 0,1 à 1 part en volume d'acide acétique pour 100 parts en volume de mélange eau / co-solvant organique polaire.

4. La solution selon une quelconque des revendications précédentes 1 à 3, dans laquelle le co-solvant organique polaire est l'acétonitrile.

5. Utilisation de la solution selon une quelconque des revendications 1 à 4 dans un procédé de fabrication d'eptifibatide qui comprend de soumettre une solution d'acétate d'eptifibatide dans un solvant à une étape de concentration, dans laquelle le pH de ladite solution est de moins que ou égal à 7.

6. Un procédé de fabrication d'eptifibatide dans lequel la solution selon une quelconque des revendications 1 à 4 est utilisée dans une étape de concentration pour obtenir un matériau d'acétate d'eptifibatide concentré.

7. Le procédé selon la revendication 6, dans lequel le matériau d'acétate d'eptifibatide concentré est une solution.

8. Le procédé selon la revendication 7, dans lequel la solution concentrée a une concentration en acétate d'eptifibatide de 4 à 5 % en poids.

9. Le procédé selon une quelconque des revendications 6 à 8, dans lequel l'étape de concentration est pratiquée à une température inférieure ou égale à 40°C.

10. Le procédé selon la revendication 9, dans lequel la température varie de 0 à 35°C.

11. Le procédé selon la revendication 10, dans lequel la température varie de 20 à 35°C.

12. Le procédé selon une quelconque des revendications 6 à 11, dans lequel l'étape de concentration comprend d'évaporer le solvant de la solution.

13. Le procédé selon la revendication 12, dans lequel le solvant est évaporé à une pression de 0,001 à 0,5 bars.

14. Solution concentrée d'acétate d'eptifibatide dans un solvant comprenant de l'eau, de l'acétonitrile et de l'acide acétique dans laquelle la concentration d'acétate d'eptifibatide dans ladite solution est égale à ou supérieure à 4 % en poids et égale à ou inférieure à 5 % en poids d'acétate d'eptifibatide, dans laquelle le pH de ladite solution concentrée d'acétate d'eptifibatide varie de 3 à 5.
